# EUROPEAN PATENT SPECIFICATION

(11) **EP 1 978 938 B1**
(45) Date of publication and mention of the grant of the patent: **09.09.2015**
(21) Application number: 07704121.8
(22) Date of filing: 24.01.2007
(51) Int. Cl.: A61K 9/16, A61K 31/192, A61K 31/337, A61K 31/573, A61K 47/44, A61K 47/32

(54) **PROCESS FOR LOADING POLYMER PARTICLES WITH DRUG**
VERFAHREN ZUM LADEN VON POLYMERTEILCHEN MIT WIRKSTOFFEN
PROCÉDÉ DE CHARGEMENT DE MÉDICAMENT DANS DES PARTICULES POLYMÈRES

(30) Priority: 24.01.2006 EP 06250387
(43) Date of publication of application: 15.10.2008
(73) Proprietor: Biocompatibles UK Limited, Farnham Surrey GU9 8QL (GB)
(72) Inventor: LEWIS, Andrew, Lennard, Farnham Surrey GU9 8QL (GB); GOREISH, Hind, Hassan, SidAhmed, Farnham Surrey GU9 8QL (GB); TANG, Yiqing, Farnham Surrey GU9 8QL (GB)
(74) Representative: BTG plc Intellectual Property Group
(86) International application number: PCT/EP2007/050690
(87) International publication number: WO 2007/085615

(56) References cited:
- WO-A-00/23054
- WO-A-01/68720
- WO-A-01/72281
- WO-A-2004/073688
- BOUDY V ET AL: "MICROENCAPSULATION DE MICROSPHERES DESTINEES A L'EMBOLISATION THERAPEUTIQUE DEFINITIVE MICROENCAPSULATION OF MICROSPHERES FOR DEFINITIVE THERAPEUTIC EMBOLIZATION" JOURNAL DE PHARMACIE CLINIQUE - INTERNATIONAL JOURNAL OF CLINICAL PHARMACY, LAVOISIER, PARIS, FR, vol. 18, no. 1, March 1999 (1999-03), pages 21-23, XP008039681 ISSN: 0291-1981
- SONG C X ET AL: "Formulation and characterization of biodegradable nanoparticles for intravascular local drug delivery" JOURNAL OF CONTROLLED RELEASE, ELSEVIER, AMSTERDAM, NL, vol. 43, no. 2-3, 18 January 1997 (1997-01-18), pages 197-212, XP004425642 ISSN: 0168-3659

## Description

The present invention relates to processes for loading polymeric beads, to be used as drug delivery media, with drugs. The processes allow loading of drugs susceptible to crystallisation within such media, whereby the burst effect is minimised. In a preferred aspect, the beads are radiopaque.

Embolisation therapy involves the introduction of an agent into the vasculature in order to bring about the deliberate blockage of a particular vessel. This type of therapy is particularly useful for blocking abnormal connections between arteries and veins (such as arteriovenous malformations, or AVMs), and also for occluding vessels that feed certain hypervascularised tumours, in order to starve the abnormal tissue and bring about its necrosis and shrinkage.

The process of embolisation may induce tumour ischemia or necrosis depending upon the extent of the embolisation. The response of the tumour cells to the hypoxic environment can result in an ensuing angiogenesis in which new blood vessels are grown to compensate for the loss of flow to the tumour by the embolisation. It would be desirable therefore to combine embolisation with the administration of agents that could prevent the ensuing angiogenic response. Moreover, it may be desirable to combine this effect with the release of a cytotoxic or other anti-tumoral agent to bring about cell death in those cells that are not killed by the embolisation. There have been many disclosures of polymer bead embolic agents loaded with therapeutic agents, for treatment of solid tumours, such as WO2004/000548, WO2005/087193, PCT/GB2004/003347 and PCT/GB2005/003431.

Oils are commonly used in pharmaceutical formulation technologies and preparations. Soya Bean oil is commonly used in the FDA Inactive Ingredients Guide (IV injections, oral capsules and topical preparations).

Lipiodol is iodinated oily X-ray contrast medium wherein poppy-seed oil is iodised to a level of about 40% by weight. It can be used in certain radiological investigations where it is desirable to outline a viscous or other structure with directly instilled radio-opaque material. It is also suitable for introduction into narrow channels and may therefore be used in embolisation procedures. It is commonly used in diagnostic hysterosalpinography (HSG). HSG is radiography (or X-ray) of the uterus and fallopian tubes used for the detection of gynaecological problems, particularly those affecting female fertility.

Lipiodol is also used for lymphography, which is the examination of the lymph nodes and lymphatic vessels, in particular for the prognostic diagnosis of leukaemia and Hodgkin's and non-Hodgkin's lymphoma. It is also used for detecting disorders of the salivary glands or ducts.

It is commonly used off-label in combination with chemotherapy i.e. treatment with a cytotoxic or other anti-tumoural agent, whereby a Lipiodol:chemo emulsion is administered intra arterially in the super-selective treatment of hepatocellular carcinoma.

Ibuprofen (IBU) is a hydrophobic drug and is known to form many types of crystals. The crystal properties of IBU are known to influence its pharmaceutical processing (Romero, A.J. et al., 1991 Pharma Acta Helv. 66(2) 34-43). The shape of IBU crystals depends on the solvent and this can be improved by the choice of a suitable solvent (Garekani HA, et al., Crystal habit modifications of IBU and their physicomechanical characteristics. Drug Dev Ind Pharm.27(8):803-9, 2001).

We have described the use of microspheres containing IBU to treat uterine fibroids in WO2004/000698.

Other groups have also described use of microspheres for encapsulating drugs. Boudy V et al. in J. Pharm Clin. 18(1) (1999) preform microspheres loaded with indomethacin and then encapsulate these with a coacervated mixture of ethylcellulose and polyisobutylene.

Song C.X. et al. in Journal of Controlled Release 43(1997) 197-212 prepare various drug-loaded poly(lactic-co-glycolic acid) nanoparticles using an emulsification/solvent evaporation technique.

However, to date the problem of drug-crystallisation during the encapsulation process has not been addressed.

Crystal formation on the embolic microspheres (MS) leads to a number of disadvantages in the manufacturing of this product. While most of the drug is up-taken inside the MS some drug remains on the surface of the MS. These crystals on the surface lead to an increased burst effect, thus resulting in an immediate dumping of a large dose of drug. The crystals also cause the beads to aggregate and clump together by hydrophobic interactions, causing them to stick to hubs of the syringes making them harder to deliver, more likely to be damaged and increasing the tendency for catheter clogging during delivery.

During the sterilisation of IBU-beads by autoclaving at 120°C. for 15 minutes, crystals appear to melt onto the bead surfaces, heating above the melting point of IBU [78°C] leading to the formation oily droplets. These droplets are responsible for a nucleation process, which leads to re-crystallisation when the vials are opened and product is exposed to air. The crystals cause the problems indicated above and lead to catheter and hub blockage during use and an increase in the burst effect of the drug.

It has been demonstrated that the physicochemical properties of IBU crystals are affected by the conditions under which the crystallisation occurs, and efforts are being made to produce compositions of IBU with excipients having the desired properties by controlling the process of crystal formation. Crystal alterations were noticed for IBU in the presence of Eudragit R S100 polymer (Kachrimanis et al., 1998 Crystallisation conditions and physicomechanical properties of IBU- Eudragit S100 spherical crystal agglomerates prepared by solvent-change technique. Int. J. Pharm. 173,61-74). Rasenack et al. (IBU crystals with optimized properties. Int. J. Pharm. 245 (1-2):9-24, 2002) have used water-soluble additives in an attempt to modify IBU crystals. They suggested that additives can interact with hydrogen bonding formed during the IBU crystallization process leading to a change in the properties of the resulting crystals. Francesco et al. (Polymethacrylates as crystallisation modifiers in monolayer transdermal patches containing IBU. E.J.P.B., 60:61-66, 2005) investigated the use of low molecular weight excipients for inhibition of crystallisation of IBU in transdermal systems to maintain efficiency and quality of the patches and prolong the shelf life of the product. They reported that the addition of propylene glycol inhibited IBU crystallisation for up to 50 days and Eudragit E (EUE) and Eudragit RL(EURL) prevented crystallisation for more than 12 months. The drying conditions or final thermal curing was reported to prevent crystallisation by Campbell et al. In US 4,832,953, a method for preventing the formation of a crystalline hydrate in a dispersion of a liquid in a non-aqueous matrix was described.

The physicochemical properties of IBU can be affected by choosing a suitable polymorphic form, by choosing a suitable crystal habit, by using special crystallization techniques, and by using a suitable preparation with excipients to modify crystal formation and retard the nucleation process. Lipids have been used as carriers for IBU in order to take advantage of the metabolic pathways of lipid biochemistry to allow for a targeted delivery and reduce ulcerogenicity (Lambert DM, 2000 Eur. J. Pharm. Sci. 11 Suppl. 2: S15-27).

According to the first aspect of the present invention there is provided a process in which swellable beads of water-insoluble, oil-insoluble polymer are contacted with a solution of a crystallisable drug in an organic solvent which is capable of penetrating the beads, whereby the drug is absorbed into the beads, characterised in that the solution further contains a crystal modifier which inhibits crystallisation of the drug, wherein the crystal modifier is miscible with the solvent, and is selected from oils, glycols and glycol ethers, and in that the polymer is anionically charged, in which the anionic groups are selected from sulphonate, phosphonate and carboxylate and the polymer is crosslinked polyvinyl alcohol.

According to a second aspect of the present invention there is provided a composition comprising swellable beads of water-insoluble, oil-insoluble polymer, a crystallisable drug and a crystal modifier which inhibits crystallisation of the drug wherein the drug and crystal modifier are absorbed into the beads, the polymer is anionically charged, in which the anionic groups are selected from sulphonate, phosphonate and carboxylate and crosslinked polyvinyl alcohol, and the crystal modifier is selected from oils, glycols and glycol ethers.

In the invention the drug which is used is generally one which is susceptible to crystallisation, for instance upon removal of solvent, usually by evaporation, or following sterilisation steps, when migration of absorbed drug to form liquid droplets at temperatures above the melting temperature, may take place forming a metastable phase susceptible to delayed crystallisation.

Crystal forming drugs are generally known to persons skilled in the art. One category of drug to which the invention is of particular value is COX (1 or 2) inhibitors, such as ibuprofen. Another class of drugs which includes such crystal forming drugs, are anti-cancer agents, for instance paclitaxel. Another class of drugs are corticosteroids, for instance having anti-inflammatory properties, one example being dexamethasone.

In the invention the polymer which forms the beads must be water-insoluble and oil-insoluble. Although it may be biodegradable, so that drug may be released substantially by erosion of polymer matrix to release drug from the surface, preferably the polymer is substantially biostable (ie non-biodegradable). The polymer is water-swellable. Water-swellable polymer useful in the invention preferably has a equilibrium water content, when swollen in phosphate buffered saline at room temperature, measured by gravimetric analysis, in the range of 40 to 99 wt%, preferably 75 to 95%. The crosslink density should be such as to allow relative movement of the polymer molecules during the swelling step. The beads may therefore be defined as swellable and at the start of the claimed process, the beads must be susceptible to at least a degree of swelling when contacted with swelling solvents. The beads may have been partially swollen before contact with the drug/solvent solution, but preferably are substantially non-swollen at the beginning of the process.

The beads are of a size to be of utility in drug delivery applications. Although very large beads, such as having a diameter more than 5 mm, may be utilised, the beads preferably have diameter of less than 2 mm, preferably up to 1500 µm. Although the process may be of utility in loading very small beads, for instance having a diameter of less than 10 µm, for instance even as low as 1 µm, such beads are hard to handle in dry form, for instance in the form in which they are swellable. Furthermore the invention is of most benefit for loading beads of utility in embolisation. As such the beads have a diameter in the range 25 to 1500 µm, preferably in the range 50 to 1200 µm, for instance in the range 100 to 1200 µm.

In the invention the term bead is intended to cover particles of all shapes, for instance rod shapes, cubes, irregular and non uniform shapes. However the invention is of most benefit where the beads are spherical, spheroidal or pellet shaped, or disk shaped. In non spherical particles, such as pellets, spheroids or disks, the maximum dimension is preferably no more than three times the minimum diameter, and preferably less than two times the minimum diameter, for instance around 1.5 or less. The size limitations mentioned above are determined by testing a sample of the swellable beads under conditions in which the beads are swollen to equilibrium in phosphate buffered saline at room temperature and the sizes are measured using an optical microscope.

The materials are of particular utility for forming compositions for use in embolisation. The compositions are preferably provided with a particle size specification which defines the spread of diameters. Preferably the beads are graded into calibrated size ranges for accurate embolisation of vessels. The particles preferably have sizes when equilibrated in phosphate buffered saline at room temperature, in the range 100 to 1500 µm, more preferably in the range 100 to 1200 µm. The calibrated ranges may comprise beads having diameters with a bandwidth of about 100 to 300 µm. The size ranges may be for instance 100 to 300 µm, 300 to 500 µm, 500 to 700 µm, 700 to 900 µm and 900 to 1200 µm.

Another type of polymer which may be used to form the water-swellable water-insoluble matrix is polyvinyl alcohol crosslinked using aldehyde-type crosslinking agents such as glutaraldehyde. For such products, the polyvinyl alcohol (PVA) may be rendered ionic by providing pendant ionic groups by reacting a functional ionic group containing compound with the hydroxyl groups. Examples of suitable functional groups for reaction with the hydroxyl groups are acylating agents, such as carboxylic acids or derivatives thereof, or other acidic groups which may form esters.

The composition according to the second aspect of this invention comprises swellable beads of water-insoluble, oil-insoluble polymer, wherein the polymer is anionically charged, in which the anionic groups are selected from sulphonate, phosphonate and carboxylate crosslinked polyvinyl alcohol.

The invention is of particular value where the polymer matrix is formed from a polyvinyl alcohol macromer, having more than one ethylenically unsaturated pendant group per molecule, by radical polymerisation of the ethylenic groups. Preferably the PVA macromer is copolymerised with ethylenically unsaturated monomers for instance including a nonionic and/or ionic monomer including anionic monomer.

The PVA macromer may be formed, for instance, by providing PVA polymer, of a suitable molecular weight such as in the range 1000 to 500,000 D, preferably 10,000 to 100,000 D, with pendant vinylic or acrylic groups. Pendant acrylic groups may be provided, for instance, by reacting acrylic or methacrylic acid with PVA to form ester linkages through some of the hydroxyl groups. Other methods for attaching vinylic groups capable of polymerisation onto polyvinyl alcohol are described in, for instance, US4,978,713 and, preferably, US5,508,317 and US5,583,163. Thus the preferred macromer comprises a backbone of polyvinyl alcohol to which is linked, via a cyclic acetal linkage, an (alk)acrylaminoalkyl moiety. Example 1 describes the synthesis of an example of such a macromer known by the approved named nelfilcon B. Preferably the PVA macromers have about 2 to 20 pendant ethylenic groups per molecule, for instance 5 to 10.

Where PVA macromers are copolymerised with ethylenically unsaturated monomers including an ionic monomer, the ionic monomer preferably has the general formula I

Y¹BQ¹ I

in which Y¹ is selected from 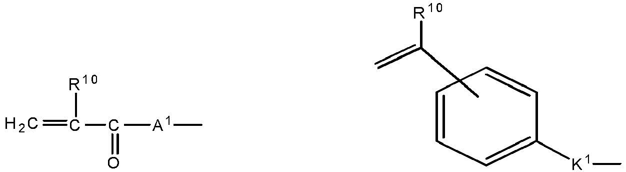 CH₂=C(R¹⁰)-CH₂-O-, CH₂=C(R¹⁰)-CH₂ OC(O)-, CH₂=C(R¹⁰)OC(O)-, CH₂=C(R¹⁰)-O-, CH₂=C(R¹⁰)CH₂OC(O)N(R¹¹)-, R¹²OOCCR¹⁰=CR¹⁰C(O)-O-, R¹⁰CH=CHC(O)O-, R¹⁰CH=C(COOR¹²)CH₂-C(O)-O-, 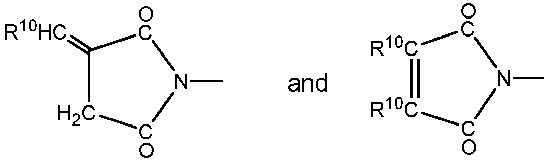 wherein:
R¹⁰ is hydrogen or a C₁-C₄ alkyl group;
R¹¹ is hydrogen or a C₁-C₄ alkyl group;
R¹² is hydrogen or a C₁₋₄ alkyl group or BQ¹ where B and Q¹ are as defined below;
A¹ is -O- or-NR¹¹-; ,
K¹ is a group -(CH₂)ᵣOC(O)-, -(CH₂)ᵣC(O)O-, - (CH₂)ᵣOC(O)O-, -(CH₂)ᵣNR¹³-,-(CH₂)ᵣNR¹³C(O)-, -(CH₂)ᵣC(O)NR¹³-, -(CH₂)ᵣNR¹³C(O)O-, -(CH₂)ᵣOC(O)NR¹³-,-(CH₂)ᵣNR¹³C(O)NR¹³- (in which the groups R¹³ are the same or different), -(CH₂)ᵣO-, -(CH₂)ᵣSO₃ -, or, optionally in combination with B, a valence bond and r is from 1 to 12 and R¹³ is hydrogen or a C₁-C₄ alkyl group;
B is a straight or branched alkanediyl, oxaalkylene, alkanediyloxaalkanediyl, or alkanediyloligo(oxaalkanediyl) chain optionally containing one or more fluorine atoms up to and including perfluorinated chains or, if Q¹ or Y¹ contains a terminal carbon atom bonded to B a valence bond; and
Q¹ is an anionic group.

An anionic group Q¹ may be, for instance, a carboxylate, sulphonate or phosphate group. The monomer may be polymerised as the free acid or in salt form. Preferably the pKₐ of the conjugate acid is less than 5.

In the monomer of general formula I preferably Y¹ is a group CH²=CR¹⁰COA¹- in which R¹⁰ is H or methyl, preferably methyl, and in which A¹ is preferably NH. B is preferably an alkanediyl group of 1 to 12, preferably 2 to 6 carbon atoms. Such monomers are acrylic monomers.

There may be included in the ethylenically unsaturated monomer diluent monomer, for instance non-ionic monomer. Such a monomer may be useful to control the pKₐ of the acid groups, to control the hydrophilicity or hydrophobicity of the product, to provide hydrophobic regions in the polymer, or merely to act as inert diluent. Examples of non-ionic diluent monomer are, for instance, alkyl (alk) acrylates and (alk) acrylamides, especially such compounds having alkyl groups with 1 to 12 carbon atoms, hydroxy, and di-hydroxy-substituted alkyl(alk) acrylates and -(alk) acrylamides, vinyl lactams, styrene and other aromatic monomers.

In the polymer matrix, the level of anion is preferably in the range 0.1 to 10 meq g⁻¹, preferably at least 1.0 meq g⁻¹. Preferred anions are derived from strong acids.

Where PVA macromer is copolymerised with other ethylenically unsaturated monomers, the weight ratio of PVA macromer to other monomer is preferably in the range of 50:1 to 1:5, more preferably in the range 20:1 to 1:2. In the ethylenically unsaturated monomer the anionic monomer is preferably present in an amount in the range 10 to 100 mole%, preferably at least 25 mole%.

The crosslinked polymer may be formed as such in particulate form, for instance by polymerising in droplets of monomer in a dispersed phase in a continuous immiscible carrier. Examples of suitable water-in-oil polymerisations to produce particles having the desired size, when swollen, are known. For instance US4,224,427 describes processes for forming uniform spherical beads (microspheres) of up to 5 mm in diameter, by dispersing water-soluble monomers into a continuous solvent phase, in a presence of suspending agents. Stabilisers and surfactants may be present to provide control over the size of the dispersed phase particles. After polymerisation, the crosslinked microspheres are recovered by known means, and washed and optionally sterilised. Preferably the particles e.g. microspheres, are swollen in an aqueous liquid, and classified according to their size.

In the method according to the first aspect of the present invention, the organic solvent is capable of dissolving the drug and penetrating the beads. In such a preferred embodiment it is selected so as to be capable of dissolving the drug. It is possible that the solvent itself acts as a crystal modifier and should hence be retained in the loaded product, in which case the solvent may be used in an amount less than required for penetrating or loading to equilibrium. However, it is preferred that the solvent is used in excess for ease of handling. After the period of contact during loading of the drug, the beads which may be swollen, and excess solvent may be separated from one another, the solvent, along with unloaded drug and excess crystallisation inhibitor. Suitable separation techniques generally involve solid-liquid separation techniques such as centrifugation and/or filtration. Generally this is followed by evaporation of further solvent, for instance by subjecting the beads to raised temperature and/or reduced pressure. Where such evaporation techniques are used, the crystal modifier must be sufficiently non-volatile such that it is not removed to a deleterious extent.

In a preferred process, the dried loaded beads, following removal of excess organic solvent, are contacted with aqueous storage liquor, preferably in an excess such that the beads are penetrated, and preferably are swollen to equilibrium and a continuous phase of extra-bead liquor is present in which the beads may be or are suspended. Preferably whilst suspended in such aqueous storage liquor, the beads are sterilised by heating the container in which the suspension is contained, for instance to a temperature of at least 90°C, more preferably at least 100°C, preferably for a period of time of at least 5 seconds, more preferably by heating at a temperature of 121°C in an autoclave, for a period of at least 15 minutes.

Preferably the crystal modifier is a liquid; it is substantially miscible with the organic solvent. Since it is preferred that excess solvent is removed by means involving evaporation, the crystal modifier is preferably substantially involatile under the solvent removal conditions. Preferably, the crystal modifier has a boiling point of more than 81 °C, more preferably a boiling point of more than 90°C. The material should also be stable under the conditions of sterilisation, where such a step is part of the process, as in the preferred embodiment. The material may also be selected having regard to its density, so that the loaded beads are isobuoyant with the storage and/or delivery medium, so that suspended in their storage solution and/or in the mixture of this suspension with contrast medium prepared immediately before delivery to a patient. In one embodiment, the density of the crystal modifier is slightly higher than that of water, for instance in the range 1.01 to 1.30 g/ml. This is the case, for instance, when Lipiodol is used as the crystal modifier. Alternatively, in a different embodiment, the crystal modifier may be less dense than water. A mixture of two or more ingredients may be used as the crystal modifier.

Examples of non-volatile miscible materials are oils or glycols or glycol ethers. One example of a glycol is glycerol. Examples of oils include soya bean oil (density 0.91-0.92 g/ml), cotton seed oil, almond oil, sunflower oil (density 0.92 g/ml), poppy seed oil and mineral oil (density 0.84-0.87 g/ml).

In a particularly preferred embodiment the crystal modifier has useful properties as an imaging agent, for instance is a radiopaque material, whereby delivery of the composition to a patient may be followed using radiographic techniques. In a highly preferred embodiment of the invention the crystal modifier is an oil based material which is radiopaque, e.g., an iodised oil, such as an iodised fatty acid lower alkyl ester, or mixture, for instance Lipiodol or Ethiodol. Lipiodol consists of iodised poppy seed oil, with some non-iodised oil stabilisier. It contains around 38 to 42% by weight iodine, partly di-iodo partly mono-iodo derivatives.

In the method of this invention the organic solvent is preferably selected from monohydric C-₁₋₁₂ aliphatic alcohols and is preferably selected from ethanol and propanol. The solvent should preferably be volatile relative to the crystal modifier. For instance it should preferably have a boiling point of less than 90°C, more preferably less than 80°C, in order to allow removal under mild conditions, e.g., involving evaporation at low temperatures and preferably low pressures.

The invention is suitable for use with a wide variety of drugs. The loading level of drug depends upon the desired dosage and may be selected by a person skilled in the art based on his knowledge of the activity of the drug, the release characteristics from the beads and the indication to be treated. Likewise the concentration of drug in the loading mixture is selected according to the desired drug loading. Preferably the concentration of drug in the loading solution is in the range 1 to 1000 mg/ml, preferably in the range 10 to 500 mg/ml. The crystal modifier should be included in the loading solution in an amount such that the end product will have a sufficient level of crystal modifier to modify the crystal formation to the desired degree. This level may be determined by a person skilled in the art by carrying out a series of tests in which different levels of drug and crystal modifier are loaded into the specific type of polymer and subjected to the process steps to which a pharmaceutical composition would be subjected, to determine the minimum level of modifier required to effect the crystallisation of the drug, that is to prevent crystallisation taking place. Suitable levels of crystal modifiers (i.e. crystallisation inhibitors) are in the range 1 to 99% w/w based on the total level of drug in the beads, preferably in the range 5 to 50%.

Suitably the drug:polymer ratio is in the range 10:1 to 1:10, based on dry matter in the beads (i.e. polymer) preferably in the range 2:1 to 1:2.

The method of this invention has been found to be of particular value for producing beads loaded with paclitaxel (PTX), ibuprofen and dexamethasone (DEX). The drug-oil loaded beads have the advantage of a reduced crystal formation on the bead surface. It is also thought that there is reduced crystal formation within the beads, which gives a more homogeneous distribution of drug in the beads. PTX-loaded beads may act as anti-tumoural embolic, whereas IBU and Dex-loaded beads may have anti-inflammatory effects as well as potentially antitumoral activity by way of their mode of action in COX inhibition. Advantages of lowering crystals formation includes: a reduced burst effect due to drug crystals on bead surface, reduced adhesive tendency for the beads to stick to packaging, such as plastics used for syringes, a reduced probability of self-aggregation in the packaging and hence the catheter and thus improved deliverability and a homogenous loading. It is additionally a possibility that the presence of the modifier may enhance the stability and thus the shelf-life of the product. The use of oil-based contrast media (iodinated oils such as lipiodol) as the crystal modifier, may impart an additional benefit in order to allow the visualisation of the beads during delivery, and potentially enabling traceability of the beads after embolisation. Iodised oils possess a greater density than water and their use as crystal modifiers also increases the overall density of the drug-loaded microspheres, such that it is possible to control the suspension properties of the beads when placed in a saline:contrast mixture typically used for the delivery of the device. Lipiodol has a density of 1.280 g/ml at 15°C.

In the invention it is preferred to use oils that are approved by the FDA as inactive ingredients, and these have been found to modify the crystal formation of ibuprofen, paclitaxel and dexamethasone. The effect on reducing aggregation of beads and adhesion to plastics used during handling and delivery is an illustration of the benefit of the invention. Furthermore the visualisation of the beads using radiography, where lipiodol is the crystal modifier, has been shown to be possible and provides a significant combination of useful properties. The use of a high density oil as the crystallisation modifier provided the further benefit that the density of the loaded beads was controlled to be closer to that of the aqueous storage medium, whereby the loaded beads could be mixed with saline and/or other contrast medium, prior to delivery without immediate settlement. In the invention therefore the product beads may be administered in usual techniques used for embolisation, including optionally suspension in additional contrast medium for additional visualisation by radiography.

The following is a brief description of the figures:
Figures 1A and 1C show Ibuprofen crystals on beads surface using Comparative Example 1;
Figures 1B and 1D show ibuprofen beads after loading using oils (Lipiodol)-Example 1;
Figure 2 shows the ibuprofen bead size distribution after loading with different concentrations of lipiodol compared to the unloaded control as described in Example 2;
Figure 3A-3E are histograms of the size distributions of ibuprofen loaded beads after hydration using different volumes of water and saline 2mL compared to the control in 0.9 %saline [w=water, PBS= phosphate buffered saline] - see Example 2;
Figure 4A shows Ibuprofen elution profile using 5% and 30% Lipiodol for the loading for beads 700-900 µm in Example 2;
Figure 4B shows Ibuprofen elution profile using 5% and 30% Lipiodol for the loading for beads 500-700 µm in Example 2;
Figure 5 illustrates the compression of Ibuprofen loaded beads using 5% and 30% and 70% Lipiodol compared to unloaded controls and beads loaded without oil (Example 3);
Figure 6A shows concentrations of ibuprofen eluted in PBS after loading using different oils (Example 6);
Figure 6B shows the % Ibuprofen eluted from beads loaded using different oils (Example 6);
Figure 7 shows the size distribution of a 500-700 µm beads after loading with ibuprofen using different oils (Example 6);
Figure 8 shows the % Ibuprofen eluted from various beads loaded with Ibuprofen-5% Lipiodol (Example 7);
Figure 9 shows various beads after 24hr elution. 9A: Bead Block IBU-5% Lipiodol. 9B: Embosphere IBU-5% lipiodol. 9C: Contour SE IBU-5% Lipiodal (Example 7);

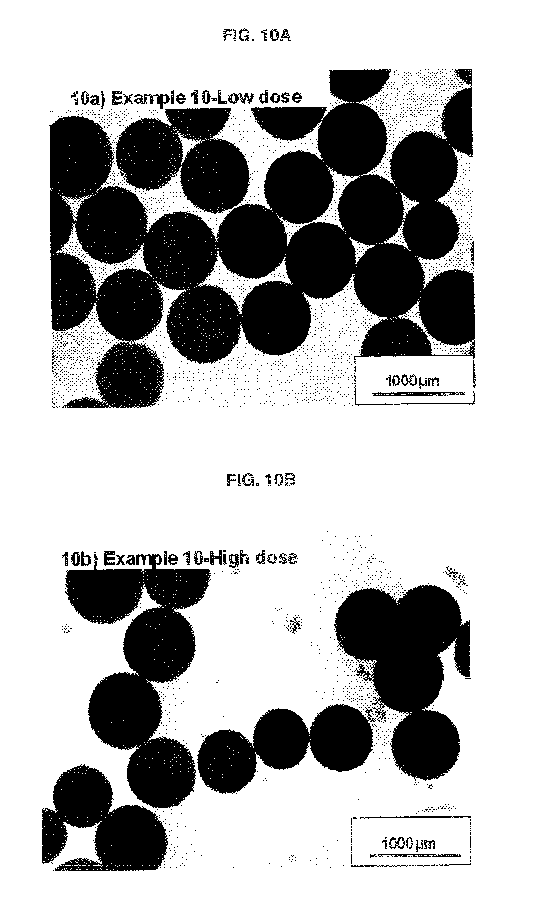

Figure 10 shows paclitaxel loaded Bead Block with Lipiodol. (A). Sample 1. (B). Sample 2 (Example 8);
Figure 11 shows dexamethasone-loaded Bead Block without Lipiodol (A) and with 10% Lipiodol (B) (Example 10);
Figure 12A shows Amberlyst-loaded with ibuprofen-Lipiodol (Example 11);
Figure 12B shows Amberlite-loaded with ibuprofen-Lipiodol (Example 11);
Figure 13 shows the release profile of paclitaxel from Bead Block loaded with Lipiodol-paclitaxel (Example 9);
Figure 14 shows the localisation of Ibuprofen Bead (IBU-BB) and Bead Block (BB) in the uterus post-embolisation (Example 12); and
Figure 15 shows the level of lymphocytes observed around Ibuprofen Bead (IBU-BB) and Bead Block (BB) at 1 week (Example 12).

The following examples illustrate the invention:
**Comparative Example: Preparation of IBU loaded Bead Block (IBU-BB)**

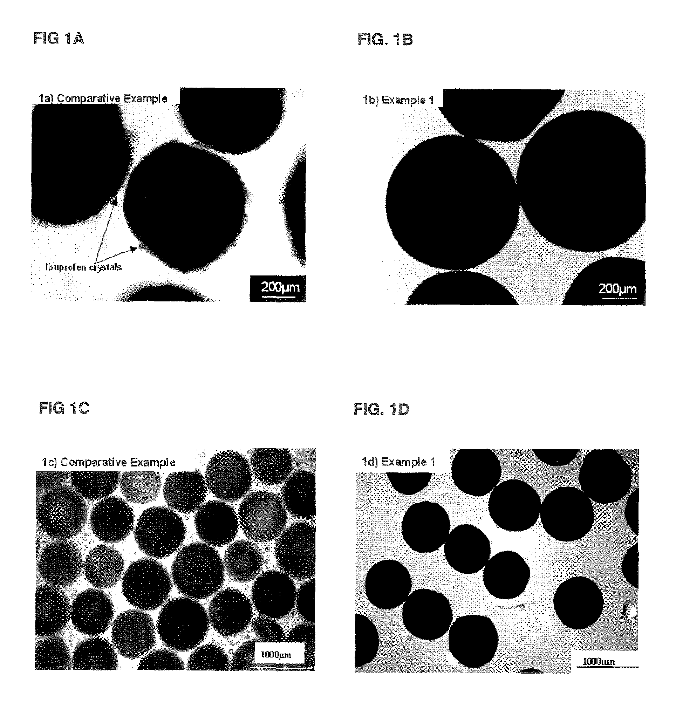

IBU-loaded beads were manufactured using the commercial available product Bead Block™ beads supplied as a sterile syringes of 2 ml beads with size range 500-700 µm. The production of such beads is described in WO2004/000548 Example 1 as the "low AMPS" product. Briefly an aqueous mixture of polyvinyl alcohol macromer having acetal-linked ethylenically unsaturated groups and 2-acrylamido-2-methyl-propane sulphonate in a weight ratio of about 12:1 is suspended in a continuous phase of butyl acetate containing cellulose acetate butyrate stabiliser with agitator and is radically polymerised using redox initiation to form beads, which are washed, dyed and sieved into size fractions including the 500-700 µm fraction and 700-900 µm fraction used in subsequent examples. Bead Block was transferred from syringes into glass vials in volumes of 2 mls per vial. The excess saline was then removed and the beads lyophilised. IBU/ethanol solution was prepared to the required final concentration. 1 ml of the solution was then added to the lyophilised beads and left to load for 1 hour (static). The excess loading solution was then removed and the bead sample dried overnight at 45°C in a vacuum oven. To the dried samples, 2 ml water was added to hydrate the beads (Figure. 1A) and they were then sterilized by autoclaving at 121°C for 15 minutes (Figure. 1C). Figure 1C shows the non-homogenous uptake of ibuprofen by beads (NB: beads with variable degrees of opacities compared to 1D) and some drug crystals in solution after loaded using Comparative Example 1.

### Example 1: Preparation of IBU-Lipiodol loaded microspheres (IBU-LBB)

The beads were loaded using the same method as in the comparative example with two exceptions. The loading solution was composed of 33% Lipiodol (L) in IBU/ethanol solution and the bead samples were shaken on a plate shaker set to 150 rpm for 1 hour. Excess loading solution was removed and samples were dried overnight in vacuum oven set to 50°C. Loaded beads were then hydrated in 2 ml water and steam sterilised as in comparative example.

The first microspheres produced using this method (IBU-LBB) were in slurry form, and were blue/white beads to the naked eye. They did not stick to the glass vials and no white crystal formation on the bead surface was apparent.

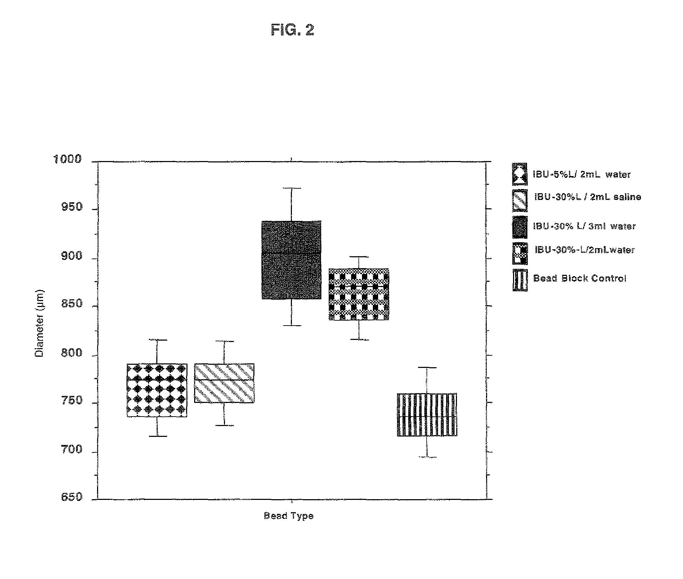

Size and Image analysis: The beads were sized using an optical microscope and visualised using a Colour View III camcorder. The beads recovered their spherical shape prior to lyophilisation, and were seen to be opaque (white/blue to the naked eye) with a homogenous appearance (Figure. 1B and 1D). The size distribution of these microspheres was comparable to the starting beads (Figure. 2).

Elution: The hydration water was removed and placed in a HPLC vial to determine the drug loss during manufacturing (neat). Each via! (2 ml of beads) was eluted into 200 ml of PBS for 24 hours. At predetermined time points (10, 20, 40, 60 minutes and 2, 4 and 24 hours), 1 ml of solution was removed and placed into HPLC vials to determine drug eluted, degradation and purity. The amount of drug loaded into the beads was similar to that without adding Lipiodol. IBU purity was >99% showing that there was no interference with IBU. Image of the beads after elution showed opaque beads that were similar in appearance to IBU loaded beads (Figure 1D). This suggests that the Lipiodol uptake by the beads contributes to the opaque colour and it does not elute in PBS.

Deliverability test: The deliverability of the beads was tested as in clinical setting for the feasibility study. The contrast medium was made by making up 10 ml of 50:50 Visipaque 320: Water. 9 ml of the mixture was added to the vials. Three injections were carried out for each sample using 3 ml and 2.7Fr Progreat® catheter.

IBU-LBB Beads did not aggregate when vials were opened and dispersed freely in saline. The deliverability test on the 700-900 µm beads demonstrated that they were deliverable from 2.7Fr Progreat microcatheter without problems.

### Example 2: Effect of hydration media, type and volume using beads loaded with IBU and 5% and 30% Lipiodol concentrations

This example was conducted to examine the degree of water uptake by beads and the effect of the hydration media used. Bead Block of size 700-900 µm was loaded using IBU ethanol solutions containing either 5% or 30% Lipiodol for 1 hour using the same general techniques Example 1. The excess loading solution was removed and samples were dried overnight in vacuum oven set at 50°C. Samples were hydrated using either 2 ml water, 3 ml water or 2 ml saline and then steam sterilized at 121°C for 15 minutes.

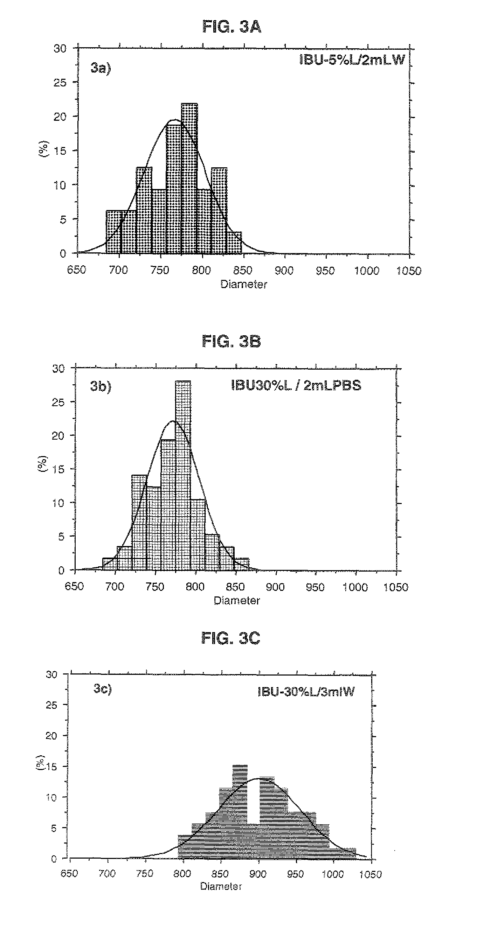

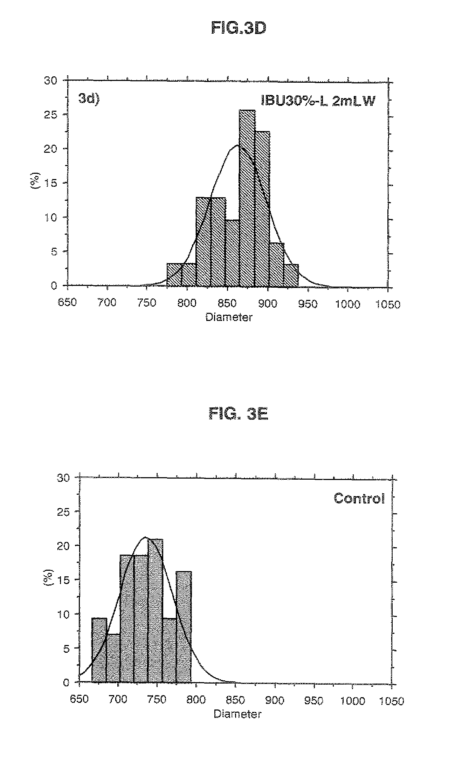

Size was measured as described in Example 1. Histograms of the size distributions are shown in Figures 3A-3E. There was a statistically significant difference (p<0.0001) between all groups compared to the control beads (prior to lyophilisation and loading). No significant difference was found between using 5% Lipiodol 2 m! water and 30% Lipiodol in 2 ml saline (p=0.5158). In both instances, the size ranges were within the acceptance criteria of the size distribution with an overall shift towards the higher beads size (Figure 2).

### Elution on IBU-5%LBB and IBU-30%LBB non beads 500-700 µm and 700-900 µm

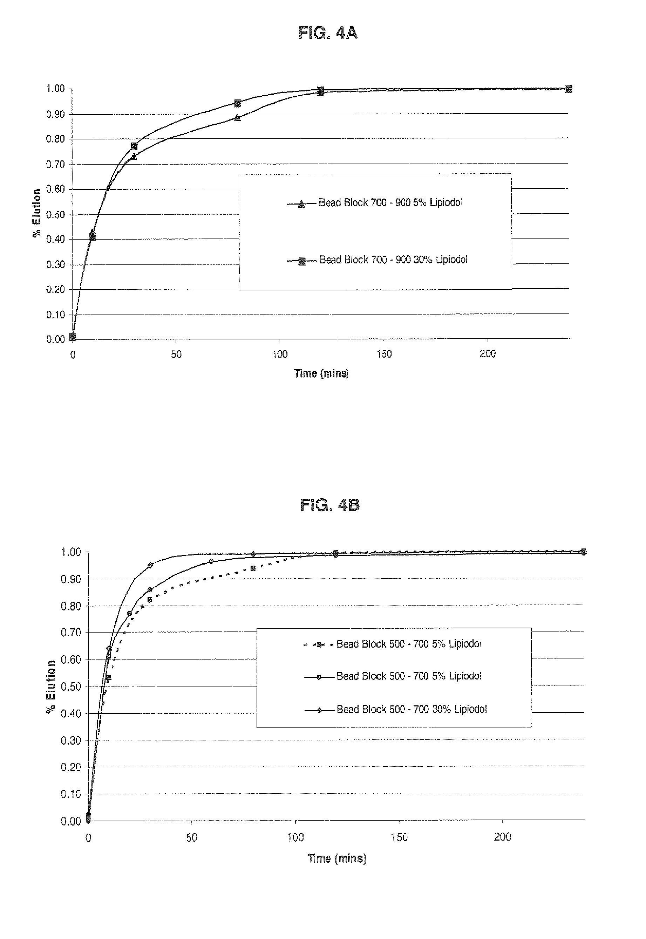

As per Example 2, the hydration water was removed and placed in a HPLC vial to determine the drug loss during manufacturing (neat). Each vial (2 ml of beads) was eluted into 200 ml of PBS for 24 hours. At predetermined time points (10, 20, 40, 60 minutes and 2, 4 and 24 hours), 1 ml of solution was removed and placed into HPLC vials to determine drug eluted, degradation and purity. Results demonstrated that for the size 700-900 µm beads there was no difference in elution rate between the beads with 5% Lipiodol or those with 30%. Figure 4A shows that for both Lipiodol concentrations, >99% of the total IBU dose is eluted in the first 2 hours for beads size 700-900 µm. Similarly for the 500-700 µm range >99% is eluted in the first 2 hours however, there was a small shift to a faster elution with the 30% Lipiodol (Figure 4B).

### Example 3: Compression testing of IBU beads containing various Lipiodol concentrations

Bead Block of size 700-900 µm was loaded IBU ethanol solutions containing: 5%, 30%, 50%, 70%, 80%, 90% Lipiodol for 1 hour. The excess loading solution was removed and samples were dried overnight in vacuum oven set at 50°C. Samples were hydrated using 2 ml water and then steam sterilized at 121°C for 15 minutes.

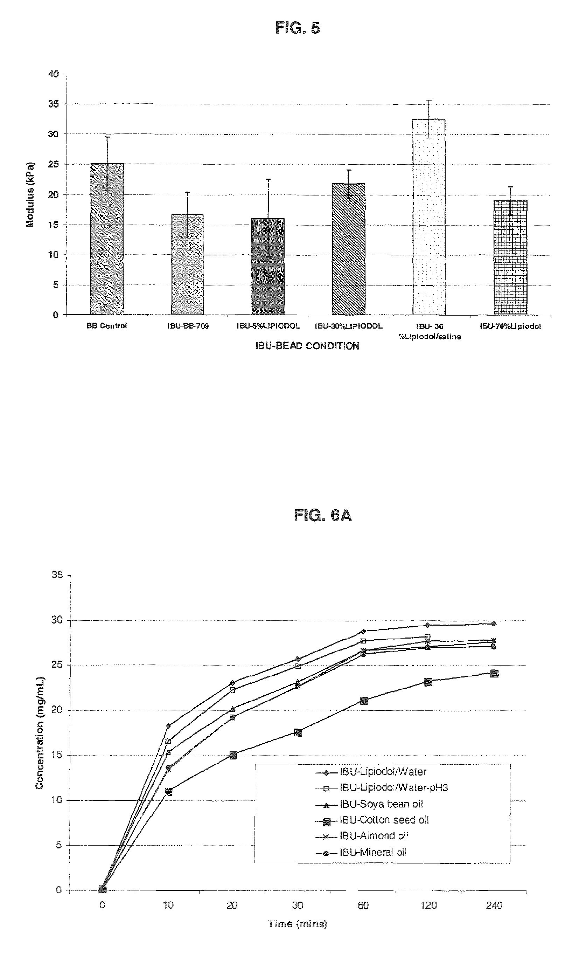

Compression testing of IBU-beads with 5%, 30% and 70% Lipiodol is shown in Figure 5, and demonstrates that IBU-LBB beads at all concentrations of Lipiodol are similar to the comparative product without Lipiodol and were all more compressible than Bead Block when it is hydrated in water. When saline was used for hydration of 30% Lipiodol IBU-LBB, the compression was higher than unloaded control.

### Example 4: Preparation of sunflower oil loaded microspheres

Bead Block was loaded with a 33% Sunflower oil/ethanol solution in place of Lipiodol using the technique described in example 1. The beads did uptake the oil and change colour to white/blue. Oily droplets were visible in the aqueous phase at this concentration. When viewed under microscope they looked similar to the IBU loaded beads (Figure. 1D).

### Example 5: Loading with different Lipiodol concentrations without drug for fluoroscopic assessment

These samples were prepared using 700-900 µm as described for the IBU/lipiodol beads above (Example 3) replacing the IBU/lipiodol solution with Lipiodol/ethanol solutions of the following concentrations: 30%, 50%, 70%, 80%, 90% Lipiodol/ethanol solutions. For the 100% Lipiodol sample these were prepared by adding Lipiodol directly into the beads for 1 hour. Then excess was removed, and then washed once quickly with ethanol. Excess ethanol was removed, dried, hydrated and steam sterilized as described for the IBU/lipiodol bead. Samples were viewed by fluoroscopy. Beads were visible under X-ray with no clear distinction between the different beads.

### Example 6: Loading IBU with different oil excipients

Bead Block of size 500-700 µm were loaded with IBU ethanol solutions containing: 5% of the following oils: Lipiodol, soyabean oil, cotton seed oil, mineral oil and almond oil for 1 hour. The excess loading solution was removed and samples were dried 2 hours in vacuum oven set at 50°C. All samples were hydrated using water and then steam sterilized at 121°C for 15 minutes.

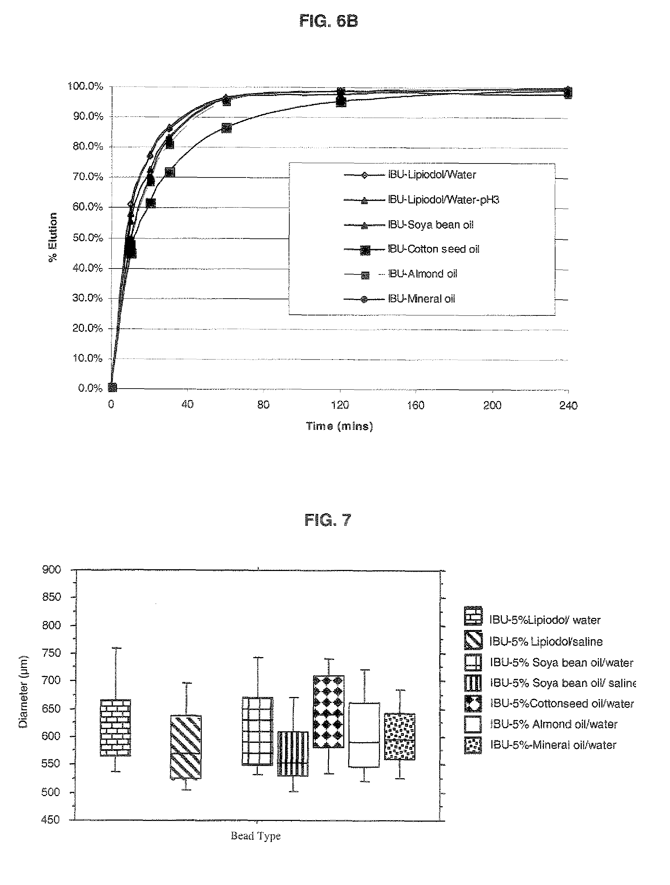

All vials looked similar to the naked eye and by image analysis, irrespective of the oil. Elution tests were carried out for all five oils as described in Example 2. The total dose eluted in 24 hours was 25 mg/ml bead ±5mg (Figure. 6A). The results showed a similar elution profile for all oils except cotton seed oil, which appeared to cause a slower rate of elution. All oils eluted 85% in the first hour except for cotton seed oil where only 72% had eluted. Within the first 4 hours, all samples had eluted >99% of the drug (Figure 6B).

The difference in bead size using all oils was not statistically significant relative to each other and to Lipiodol (Figure 7).

### Example 7: Loading other commercially available embolic beads

Commercially available embolic beads including: Embosphere (a trisacryl/gelatin microsphere coated with collagen 700-900 µm diameter product), Embogold (300 to 500 µm diameter product), Contour SE (a non-ionic cross-linked polyvinyl alcohol, 500 to 700 µm diameter product) microsphere were loaded with IBU using one loading solution of IBU/ethanol/5% Lipiodol (125 mg/mL). The loading procedure is as described in Example 2.

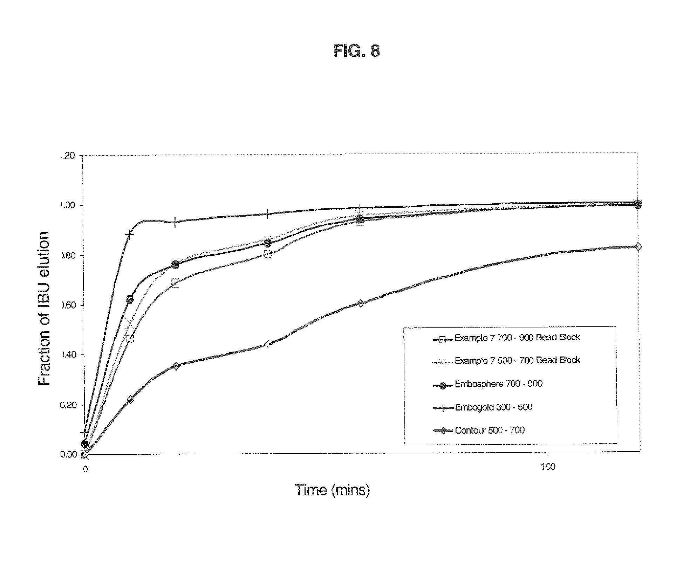

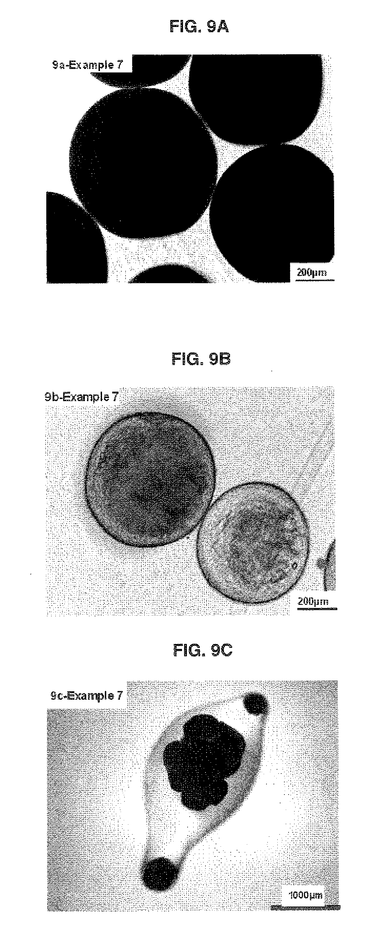

Visually, beads did not change colour after loading except for Bead Block, which changed to the typical blue/white colour. Embosphere showed high tendency to stick to the glass vial whereas Contour SE showed a great deal of aggregation. Both stickiness and aggregation are associated with crystals of IBU on the surface of the beads. Images after elution have shown that Embosphere was slightly opaque (Figure 9B) and Embogoid was clear red (compared to very opaque Bead Block (Figure 9A)) indicating lower uptake of Lipiodol and hence reduced inhibition for crystal formation. Contour SE was opaque but most beads were in clumps enclosed in oil capsule (Figure 9C). This suggests that the bead opaque colour was due to IBU uptake. The aggregation may have been caused by IBU on the bead surface following the lyophilisation stage. The enclosure of Contour SE by Lipiodol was demonstrated by the slow elution of IBU (Figure 8), whereas all other bead types eluted >98% IBU in first 2 hours.

### Example 8: Paclitaxel (PTX) loading into Lipiodol Bead Black

PTX-loaded beads were prepared using the same method described in Example 2. Bead Block (500-700 µm and 700-900 µm) was first partitioned in 2 ml into glass vials. The excess saline was then removed, and the beads were lyophilised. PTX-Lipiodol-ethanol solution was prepared to the required final concentration, and subsequently added to the lyophilised beads and left to load for 1 hour on a plate shaker with 150 rpm. The excess loading solution was then removed and the bead sample dried overnight at 45°C in a vacuum oven. To the dried samples, 2 ml water was added to hydrate the beads and they were then steam-sterilized by autoclaving at 121 °C for 15 minutes.

The PTX loading content was determined by using DMSO to extract loaded PTX under ultrasonication. The extracted solution was examined by HPLC, and the results are given in Table 1 below.

**Table 1: PTX loading efficacy in Lipiodol Bead Block ***

| Sample | Target loading (mg) | Actual loading (mg) | Loading efficacy (%) |
|---|---|---|---|
| 1 (500-700 µm) | 21.4 | 2.2 | 10.2 |
| 2 (500-700 µm) | 38.8 | 6.3 | 16.4 |
| 3 (700-900 µm) | 21.0 | 7.4 | 35.2 |
| 4 (700-900 µm) | 48.8 | 16.7 | 34.2 |

| | | | |
|---|---|---|---|
| * Lipiodol is 23 % (v/v) in loading solution | | | |

### Example 9: Elution of PTX from Lipiodol Bead Block

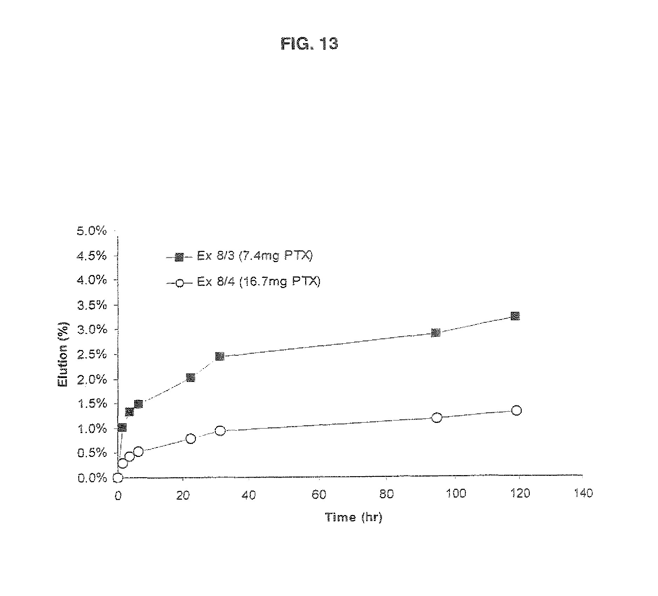

Sample 3 and 4 from Example 8 were used for the PTX elution test. 1.2 ml of each sample was mixed with 200 ml PBS buffer on a roller mixer under room temperature. At predetermined time interval, 50 or 100 ml solution was removed and 50 or 100 ml fresh PBS was added. The elution profile after HPLC processing is shown in Figure 13.

### Example 10: Loading and elution of Dexamethasone (Dex) using Lipiodol

Dex loading solution (5mg/ml) in ethanol was prepared. Using this solution beads were loaded as described in Example 1. As a control, additional beads were loaded with Dex ethanol solution without the use of Lipiodol.

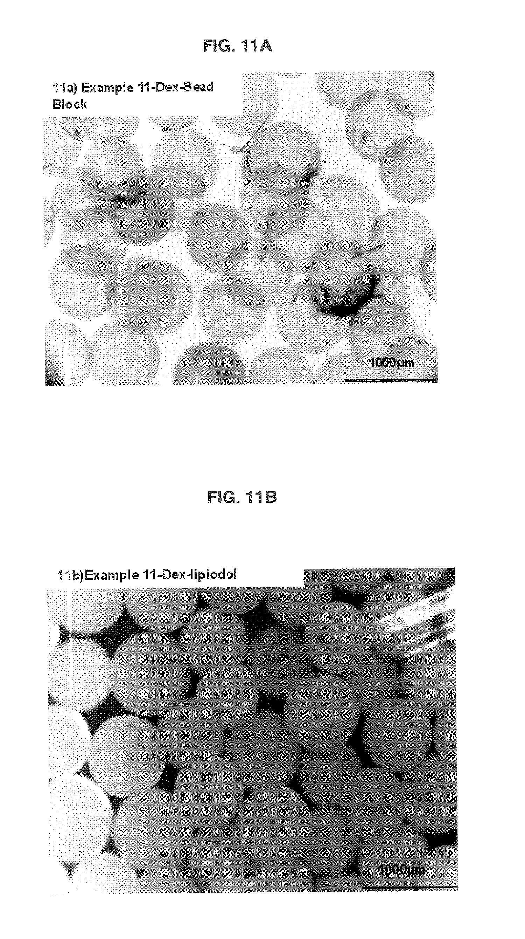

Images showed that samples loaded without the use of Lipiodol did not uptake Dex. Crystals of Dex were present on the beads surface (Figure 11 A). Beads loaded using IBU-Lipiodol were opaque and no crystals were apparent on beads surface (Figure 11B).

### Example 11: Loading and elution using different beads

Two types of ion-exchange resins (Amberlyst (SIGMA, sulphonic acid based, 1.9 mg/ml exchange capacity) and Amberlite (Sigma, IRA700 (chloride) quaternary ammonium-group containing styrene-DVB resin having total exchange capacity of 1.4 mg/ml, density 0.7 g/ml)) were loaded with IBU-Lipiodol. The loading and elution procedure was as described in Example 1 using a solution containing 100 mg/mL IBU.

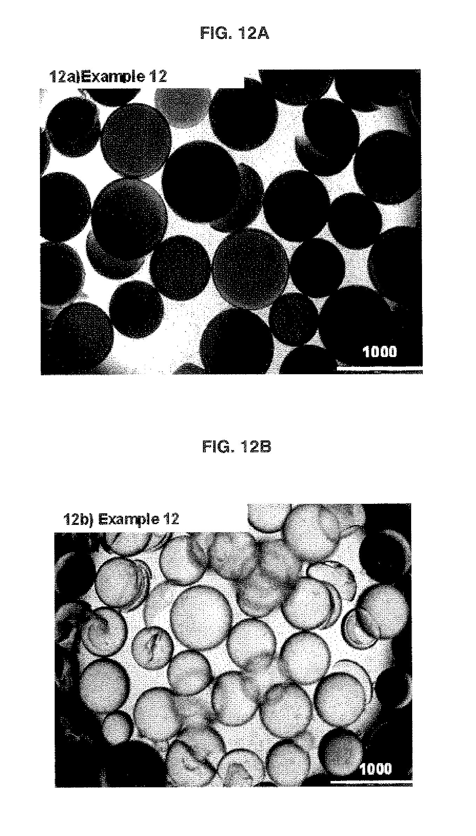

Amberlite uptake was <1 mg/mL bead of IBU (Figure 12B). Amberlyst was loaded with IBU ∼15 mg/mL (Figure 12A).

### Example 12: Use of an Embolisation Bead Containing Ibuprofen and Soya Bean Oil as A Crystallisation Inhibitor in a Sheep Uterine Artery Embolization Model of Inflammation

The effect of Ibuprofen loaded Beads was evaluated over a 12-week period post-embolisation of the uterine artery of sheep. The embolisation of blood vessels may result in a variety of side effects which can include pain and inflammation. The objective of this Example was to assess the release and effect of Ibuprofen from Ibuprofen loaded Beads containing soya bean oil as the crystallization inhibitor on the foreign body inflammatory reaction in a sheep uterine artery model. The following were assessed:
- Properties and distribution of beads within the vascular network.
- Effect of Ibuprofen / soya bean oil release on the foreign body inflammatory reaction to the embolic beads.
- Level of Ibuprofen present in the bead over time.

### Methods:

The test article in this Example was Ibuprofen loaded Beads ("Ibruprofen Bead") containing soya bean oil as crystallisation inhibitor as described in Example 6. Samples were tested against a manufacturing control of unloaded Bead Block microspheres that had been processed in a similar way to Ibuprofen Bead. 2 ml of beads were lypholised as described in Comparative Example 1 and place in 1 ml of ethanol containing 10% v/v soya bean oil and 400 mg of ibuprofen. After processing as described in Example 6, the final product contained 125 mg ibuprofen/ml hydrated beads and -10 mg of soya bean oil as determined by extraction and HPLC analysis.

Twenty four non-pregnant hormonally prepared adult Pre-Alpes sheep were divided into 2 groups, unloaded Bead Block (control, BB; 500-700µm) and Ibuprofen loaded Beads (test group, Ibuprofen Bead; 500-700µm). Each group was split into 4 time points (24 h, week 1, week 3 and week 12) with 3 animals per time point.

Embolisation was performed using the same sheath, catheters and guide wires as used in clinical practice. A 4-Fr vascular sheath was placed in the femoral artery by means of the standard Seldinger technique under sterile conditions. A 4-Fr pigtail catheter (Optitorque Radifocus; Terumo) was placed at the level of the iliac bifurcation to perform digital subtraction aortography and to identify the ovarian and uterine arteries. Selective catheterisation of the contralateral internal iliac artery and super selective catheterisation of the uterine artery was performed by using a 4-Fr cobra shaped catheter (Radiofocus Angiographic catheter, Terumo). The radiologist then performed the embolisation using the selected embolic agent (Ibuprofen Bead or Bead Block) according the randomisation sequence.

A volume of 0.5 mL of beads, suspended in a 50/50 mixture of Vispaque 270 and saline at a dilution of 1/10, was slowly injected under fluoroscopic control. Embolisation was considered complete when 0.5 mL of beads was delivered into each uterine artery.

The distribution of the beads in the tissue was assessed and gross examination of the organs performed. Inflammation was estimated histologically by semi-quantitative classification of lymphocytes by staining and use of videoanalysis post immunohistolabeling with CD-antibodies to a variety of inflammatory cells.

### Results:

### Embolisation procedure

Bead delivery was excellent in all animals. Embolisation was successful in all animals. Each animal received a total of 0.5 mL of beads in each uterine artery (total 1 mL).

### Bead performance

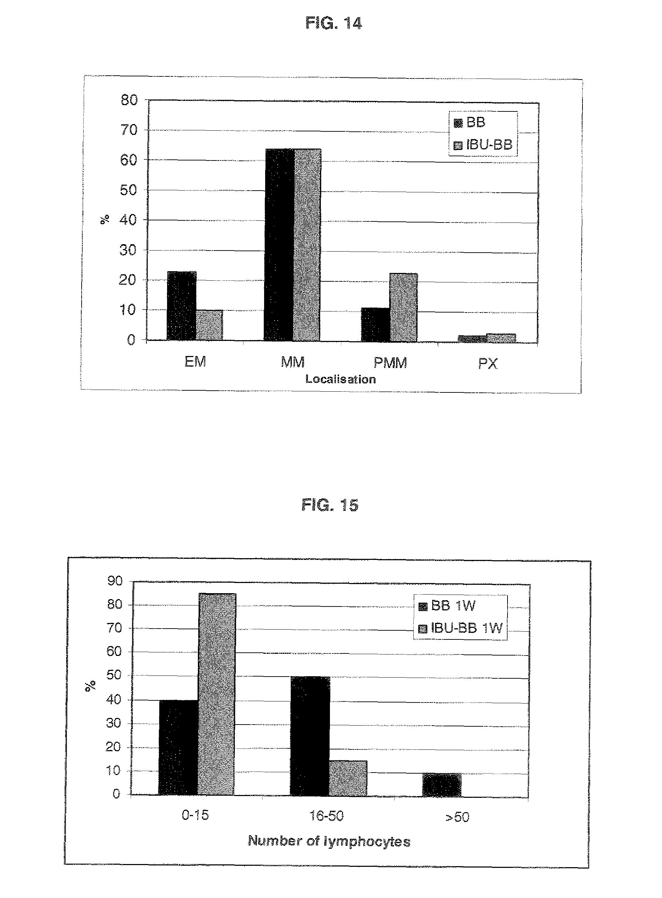

Localisation of embolised vessels in the different uterine zones was statistically different between the 2 products: Ibuprofen Bead occluded slightly more proximal vessels than Bead Block (p<0.0001, X²) (Figure 14). In Figure 14 EM stands for endometrium, MM for myometrium, PMM for perimyometrium and PX for proximal. The vessel diameter was increased for Ibuprofen Bead (498 µm vs. 436 µm, p=0.0001), however there was no difference in the mean number of beads found in embolised vessels.

More vessel necrosis was observed with Ibuprofen Bead than Bead Block at 24 hours (25.8% vs. 4.9%) and 1 week (32.8% *vs.* 4.3%) post-embolisation, however at 3 weeks and 3 months no necrosis was found for either group.

### Inflammatory response

Minor differences in numbers of neutrophils and eosinophils were observed for Bead Block and Ibuprofen Bead at certain time points. There were statistically less giant cells for Ibuprofen Bead than Bead Block at 1 week (p =0.01). At 24 hours few lymphocytes were observed in both groups, and levels were not significantly different. At 1 week, the number of lymphocytes was significantly lower around Ibuprofen Bead compared to Bead Block (Figure 15). At 3 weeks and 3 months the number of lymphocytes with Ibuprofen Bead increased, but was not significantly different from Bead Block.

In some of the histological slides there were signs of resorption of both BB and Ibuprofen Bead products, with some apparent phagocytosis, increased presence of giant cells and continued presence of CD4 and CD8. This is not unexpected given previous long-term observations with PVA embolisation agents.

### CD markers

The reduction in inflammatory response was confirmed by reduced levels of CD172a-positive cells in the Ibuprofen Bead group at week 1. Further, the addition of a crystallisation inhibitor did not result in infiltration of CD8 positive T cells or CD21 B cells, indicating no adverse reaction to the optimised formulation of Ibuprofen Bead. The other CD markers did not show any difference between the two groups. The study did confirm however, that there was no "rebound effect" at 3 months, with CD172, CD3 and MHC-II all remaining low, and no signs of hypersensitivity with absence of CD21+ or eosinophilic cells.

### Ibuprofen levels

Areas of specific staining with the anti-Ibuprofen antibody were identified as those with a "reticule" pattern. High levels of staining were observed at 24 hours post-embolisation. Ibuprofen was still detectable at week 1, but at much lower levels. No Ibuprofen was detected in the beads at 3 week and 3 months post-embolisation.

### Conclusions

This Example confirms that embolisation with Ibuprofen Bead can reduce the foreign body inflammatory response to the embolic beads. Ibuprofen can be detected in the beads up to one week post-embolisation but not at 3 weeks or beyond. Further, the reduction in the inflammatory response at 1 week does not result in any adverse rebound-effect on the inflammatory response at 3 weeks and 3 months post-embolisation. There was no obvious in vivo effect of the inclusion of the soya bean oil crystallization inhibitor.

## Claims

1. A process in which swellable beads of water-insoluble, oil-insoluble polymer are contacted with a solution of a crystallisable drug in an organic solvent which is capable of penetrating the beads whereby drug is absorbed into the beads, **characterised in that** the solution further contains a crystal modifier which inhibits crystallisation of the drug, wherein the crystal modifier is miscible with the solvent and is selected from oils, glycols and glycol ethers, and **in that** the polymer is anionically charged, in which the anionic groups are selected from sulphonate, phosphonate and carboxylate and the polymer is crosslinked polyvinyl alcohol.

2. A process according to claim 1, in which excess solvent is removed from the beads to produce dried loaded beads.

3. A process according to claim 2, in which the dried loaded beads are contacted with excess aqueous storage liquor to swell them to equilibrium and are then sterilised by heating.

4. A process according to any preceding claim, wherein the crystal modifier has a boiling point of more than 81 °C.

5. A process according to claim 1, in which the crystal modifier is an oil selected from soya bean oil, poppy seed oil, sunflower oil and mineral oil.

6. A process according to claim 5, in which the oil is iodinated.

7. A process according to any preceding claim, in which the organic solvent is a volatile solvent, having a boiling point of less than 90°C.

8. A process according to claim 7, in which the solvent is selected from monohydric C₁₋₁₂-aliphatic alcohols.

9. A process according to any preceding claim, in which the concentration of drug in the solution is in the range 1 to 1000 mg/ml.

10. A process according to any preceding claim, in which the concentration of crystal modifier in the solution is in the range 1 to 99%w/w based on drug.

11. A process according to any preceding claim, in which the polymer is formed by copolymerising ethylenically unsaturated polyvinyl alcohol macromer with ethylenically unsaturated anionic comonomer.

12. A process according to any preceding claim, in which at the start of the process the beads have sizes such that, when swollen to equilibrium in phosphate buffered saline at 20°C, the average diameter is in the range 50 to 1500 µm.

13. A process according to any preceding claim, in which the drug is selected from chemotherapeutics, anti-inflammatories, steroids and analgesics.

14. A process according to claim 13, in which the drug is selected from paclitaxel, ibuprofen and dexamethasone.

15. A composition comprising swellable beads of water-insoluble, oil-insoluble polymer, a crystallisable drug and a crystal modifier which inhibits crystallisation of the drug wherein the drug and crystal modifier are absorbed into the beads, the polymer is anionically charged, in which the anionic groups are selected from sulphonate, phosphonate and carboxylate, and is crosslinked polyvinyl alcohol and the crystal modifier is selected from oils, glycols and glycol ethers.

16. A composition according to claim 15, in which the crystal modifier is an oil or glycol.

17. A composition according to claim 16, wherein the crystal modifier is an oil selected from soya bean oil, poppy seed oil, sunflower oil and mineral oil.

18. A composition according to claim 17, wherein the oil is iodinated.

19. A composition according to any of claims 15 to 18, which is sterile and further comprises excess aqueous storage liquor with which the beads are in contact, wherein the beads are swelled to equilibrium.

20. A composition according to any of claims 15 to 19, wherein the drug:polymer weight ratio is in the range 10:1 to 1:10.

21. A composition according to any of claims 15 to 20, wherein the crystal modifier is present in the range 1 to 99% w/w based on the total level of the drug in the beads.

22. A composition according to claim 15, wherein the polymer has the further features defined in claim 11, and the drug has the further features defined in claim 13 or 14.

## Patentansprüche

1. Prozess, bei dem quellbare Kügelchen eines wasserunlöslichen, nicht in Öl löslichen Polymers mit einer Lösung eines kristallisierbaren Arzneimittels in einem organischen Lösungsmittel, das zum Durchdringen der Kügelchen in der Lage ist, in Berührung gebracht werden, wodurch das Arzneimittel in die Kügelchen absorbiert wird, **dadurch gekennzeichnet, dass** die Lösung ferner einen Kristallmodifikator enthält, welcher eine Kristallisation des Arzneimittels hemmt, wobei der Kristallmodifikator mit dem Lösungsmittel mischbar ist und aus Ölen, Glycolen und Glycolethern ausgewählt ist, und dadurch, dass das Polymer anionisch geladen ist, wobei die anionischen Gruppen aus Sulfonat, Phosphonat und Carboxylat ausgewählt sind, und es sich bei dem Polymer um vernetzten Polyvinylalkohol handelt.

2. Prozess nach Anspruch 1, bei dem überschüssiges Lösungsmittel von den Kügelchen entfernt wird, um getrocknete beladene Kügelchen herzustellen.

3. Prozess nach Anspruch 2, bei dem die getrockneten beladenen Kügelchen mit einem Überschuss an wässriger Einlagerungsflüssigkeit in Berührung gebracht werden, um sie zu quellen, bis ein Gleichgewicht erreicht ist, und dann durch Erhitzen sterilisiert werden.

4. Prozess nach einem der vorhergehenden Ansprüche, wobei der Kristallmodifikator einen Siedepunkt von mehr als 81 °C aufweist.

5. Prozess nach Anspruch 1, bei dem es sich bei dem Kristallmodifikator um ein Öl handelt, das aus Sojaöl, Mohnöl, Sonnenblumenöl und Mineralöl ausgewählt ist.

6. Prozess nach Anspruch 5, bei dem das Öl iodiert ist.

7. Prozess nach einem der vorhergehenden Ansprüche, bei dem es sich bei dem organischen Lösungsmittel um ein flüchtiges Lösungsmittel handelt, welches einen Siedepunkt von weniger als 90 °C aufweist.

8. Prozess nach Anspruch 7, bei dem das Lösungsmittel aus einwertigen aliphatischen C₁-C₁₂-Alkoholen ausgewählt ist.

9. Prozess nach einem der vorhergehenden Ansprüche, bei dem die Konzentration des Arzneimittels in der Lösung im Bereich von 1 bis 1000 mg/ml liegt.

10. Prozess nach einem der vorhergehenden Ansprüche, bei dem die Konzentration des Kristallmodifikators in der Lösung im Bereich von 1 bis 99 % Gew./Gew. auf Basis des Arzneimittels liegt.

11. Prozess nach einem der vorhergehenden Ansprüche, bei dem das Polymer durch Copolymerisieren eines ethylenisch ungesättigten Polyvinylalkohol-Makromers mit einem ethylenisch ungesättigten anionischen Comonomer gebildet ist.

12. Prozess nach einem der vorhergehenden Ansprüche, bei dem die Größen der Kügelchen zu Beginn des Prozesses derart sind, dass der durchschnittliche Durchmesser im Bereich von 50 bis 1500 µm liegt, wenn sie in phosphatgepufferter Salzlösung bei 20 °C gequollen sind, bis ein Gleichgewicht erreicht ist.

13. Prozess nach einem der vorhergehenden Ansprüche, bei dem das Arzneimittel aus Chemotherapeutika, entzündungshemmenden Stoffen, Steroiden und Analgetika ausgewählt ist.

14. Prozess nach Anspruch 13, bei dem das Arzneimittel aus Paclitaxel, Ibuprofen und Dexamethason ausgewählt ist.

15. Zusammensetzung, welche quellbare Kügelchen eines wasserunlöslichen, nicht in Öl löslichen Polymers, ein kristallisierbares Arzneimittel und einen Kristallmodifikator aufweist, welcher eine Kristallisation des Arzneimittels hemmt, wobei das Arzneimittel und der Kristallmodifikator in die Kügelchen absorbiert sind, das Polymer anionisch geladen ist, wobei die anionischen Gruppen aus Sulfonat, Phosphonat und Carboxylat ausgewählt sind, und es sich dabei um vernetzten Polyvinylalkohol handelt und der Kristallmodifikator aus Ölen, Glycolen und Glycolethern ausgewählt ist.

16. Zusammensetzung nach Anspruch 15, wobei es sich bei dem Kristallmodifikator um ein Öl oder Glycol handelt.

17. Zusammensetzung nach Anspruch 16, wobei es sich bei dem Kristallmodifikator um ein Öl handelt, das aus Sojaöl, Mohnöl, Sonnenblumenöl und Mineralöl ausgewählt ist.

18. Zusammensetzung nach Anspruch 17, wobei das Öl iodiert ist.

19. Zusammensetzung nach einem der Ansprüche 15 bis 18, die steril ist und ferner einen Überschuss an wässriger Einlagerungsflüssigkeit aufweist, mit der die Kügelchen in Berührung sind, wobei die Kügelchen gequollen werden, bis ein Gleichgewicht erreicht ist.

20. Zusammensetzung nach einem der Ansprüche 15 bis 19, wobei das Gewichtsverhältnis von Arzneimittel zu Polymer im Bereich von 10 zu 1 bis 1 zu 10 liegt.

21. Zusammensetzung nach einem der Ansprüche 15 bis 20, wobei der Kristallmodifikator im Bereich von 1 bis 99 % Gew./Gew. auf der Basis der Gesamtmenge des Arzneimittels in den Kügelchen vorhanden ist.

22. Zusammensetzung nach Anspruch 15, wobei das Polymer die weiteren Merkmale aufweist, die in Anspruch 11 definiert sind, und das Arzneimittel die weiteren Merkmale aufweist, die in Anspruch 13 oder 14 definiert sind.

## Revendications

1. Procédé dans lequel des billes gonflables d'un polymère insoluble dans l'eau et insoluble dans l'huile sont mises en contact avec une solution d'un médicament cristallisable dans un solvant organique qui est capable de pénétrer les billes, moyennant quoi le médicament est absorbé dans les billes, **caractérisé en ce que** la solution contient en outre un agent modificateur de cristal qui inhibe la cristallisation du médicament, dans lequel l'agent modificateur de cristal est miscible avec le solvant et est sélectionné parmi des huiles, glycols et éthers de glycol, et **en ce que** le polymère est anioniquement chargé, les groupes anioniques étant sélectionnés parmi le sulfonate, le phosphonate et le carboxylate, et le polymère est un alcool polyvinylique réticulé.

2. Procédé selon la revendication 1, dans lequel l'excès de solvant est éliminé des billes pour produire des billes chargées séchées.

3. Procédé selon la revendication 2, dans lequel les billes chargées séchées sont mises en contact avec une liqueur de stockage aqueuse en excès pour les gonfler à l'équilibre, puis sont stérilisées par chaleur.

4. Procédé selon l'une quelconque des revendications précédentes, dans lequel l'agent modificateur de cristal a un point d'ébullition supérieur à 81 °C

5. Procédé selon la revendication 1, dans lequel l'agent modificateur de cristal est une huile choisie parmi l'huile de soja, l'huile de graines de pavot, l'huile de tournesol et une huile minérale.

6. Procédé selon la revendication 5, dans lequel l'huile est iodée.

7. Procédé selon l'une quelconque des revendications précédentes, dans lequel le solvant organique est un solvant volatile ayant un point d'ébullition inférieur à 90 °C.

8. Procédé selon la revendication 7, dans lequel, le solvant est sélectionné parmi les alcools aliphatiques en C₁₋₁₂ monohydriques.

9. Procédé selon l'une quelconque des revendications précédentes, dans lequel la concentration du médicament dans la solution est dans la plage de 1 à 1000 mg/ml.

10. Procédé selon l'une quelconque des revendications précédentes, dans lequel la concentration de l'agent modificateur de cristal dans la solution est dans la plage de 1 à 99 % en poids par rapport au médicament.

11. Procédé selon l'une quelconque des revendications précédentes, dans lequel le polymère est formé en copolymérisant un macromère alcool polyvinylique éthyléniquement insaturé avec un comonomère anionique éthyléniquement insaturé.

12. Procédé selon l'une quelconque des revendications précédentes, dans lequel au démarrage du procédé les billes ont des tailles telles que, lorsqu'elles sont gonflées à l'équilibre dans une solution saline tamponnée au phosphate à 20 °C, le diamètre moyen est dans la plage de 50 à 1500 µm.

13. Procédé selon l'une quelconque des revendications précédentes, dans lequel le médicament est sélectionné parmi des agents chimio-thérapeutiques, des anti-inflammatoires, des stéroïdes et des analgésiques.

14. Procédé selon la revendication 13, dans lequel le médicament est sélectionné parmi le paclitaxel, l'ibuprofène et le dexaméthasone.

15. Composition comprenant des billes gonflables d'un polymère insoluble dans l'eau et insoluble dans l'huile, un médicament cristallisable et un agent modificateur de cristal qui inhibe la cristallisation du médicament, moyennant quoi le médicament et l'agent modificateur de cristal sont absorbés dans les billes, le polymère est anioniquement chargé, les groupes anioniques étant sélectionnés parmi le sulfonate, le phosphonate et le carboxylate, et est un alcool polyvinylique réticulé, et l'agent modificateur de cristal est choisi parmi des huiles, des glycols et des éthers de glycol.

16. Composition selon la revendication 15, dans laquelle l'agent modificateur de cristal est une huile ou un glycol.

17. Composition selon la revendication 16, dans laquelle l'agent modificateur de cristal est une huile sélectionnée parmi l'huile de soja, l'huile de graines de pavot, l'huile de tournesol et une huile minérale.

18. Composition selon la revendication 17, dans laquelle l'huile est iodée.

19. Composition selon l'une quelconque des revendications 15 à 18, qui est stérile et comprend en outre une liqueur de stockage aqueuse en excès avec laquelle les billes sont en contact, dans laquelle les billes sont gonflées à l'équilibre.

20. Composition selon l'une quelconque des revendications 15 à 19, dans laquelle le rapport en poids médicament:polymère est dans la plage 10:1 à 1:10.

21. Composition selon l'une quelconque des revendications 15 à 20, dans laquelle l'agent modificateur de cristal est présent dans la plage 1 à 99 % en poids par rapport au niveau total de médicament dans les billes.

22. Composition selon la revendication 15, dans laquelle le polymère possède les propriétés supplémentaires définies dans la revendication 11, et le médicament possède les propriétés supplémentaires définies dans la revendication 13 ou 14.
